(19) Europäisches Patentamt
European Patent Office
Office européen des brevets

(11) EP 4 736 834 A1

(12) EUROPEAN PATENT APPLICATION
published in accordance with Art. 153(4) EPC

(43) Date of publication:
**06.05.2026 Bulletin 2026/19**

(21) Application number: **24831800.8**

(22) Date of filing: **19.06.2024**

(51) International Patent Classification (IPC):
***A61K 6/20*** *(2020.01)* ***A61K 8/19*** *(2006.01)*
***A61K 8/27*** *(2006.01)* ***A61K 8/55*** *(2006.01)*
***A61Q 11/00*** *(2006.01)*

(52) Cooperative Patent Classification (CPC):
**A61K 6/20; A61K 8/19; A61K 8/27; A61K 8/55; A61Q 11/00**

(86) International application number:
**PCT/JP2024/022269**

(87) International publication number:
**WO 2025/004938 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.06.2023 JP 2023108725**

(71) Applicant: **Kuraray Noritake Dental Inc.**
**Kurashiki-shi, Okayama 710-0801 (JP)**

(72) Inventors:
- **NOJIRI, Yamato**
  **Tokyo 100-0004 (JP)**
- **ISHIGURO, Eri**
  **Tainai-shi, Niigata 959-2653 (JP)**
- **KAWASHIMA, Mitsunobu**
  **Tainai-shi, Niigata 959-2653 (JP)**

(74) Representative: **D Young & Co LLP**
**3 Noble Street**
**London EC2V 7BQ (GB)**

(54) **DENTAL COMPOSITION KIT**

(57) A dental composition kit having a high mineral density restoration capability is provided, the dental composition kit including a liquid A and a liquid B, the liquid A containing a calcium ion (a1) and water (b1), the liquid B containing a compound (c) having an acidic group represented by the following formula (I):

$$R^1\text{-}X_k \qquad \text{Formula (I)}$$

in which in the formula (I), $R^1$ represents an organic group having 40 or less carbon atoms having or not having at least one (meth)acryloyloxy group; X represents an acidic group; and k represents an integer of 1 or 2, in which in the case where k represents 2, two acidic groups X are the same as or different from each other, a zinc ion (d1), and water (b2), containing substantially no crosslinking polymerizable monomer.

EP 4 736 834 A1

## Description

### Technical Field

The present invention relates to a dental composition kit.

### Background Art

A dental composition having a metal salt blended therein, and a dental composition kit for coating two kinds of compositions sequentially on a tooth surface have been proposed.

For example, PLT 1 discloses anti-hyperesthesia agent containing a zinc salt, an aluminum salt, and an anionic surfactant.

PTL 2 discloses a dental treating material for relieving dental hypersensitivity, including two liquids A and B containing substances capable of forming a difficultly soluble precipitate immediately through mixing, which are coated sequentially on a tooth surface to form precipitate.

### Citation List

### Patent Literatures

PTL 1: JPH 03-258724 A
PTL 2: JPH 04-217904 A

### Summary of Invention

### Technical Problem

Incidentally, on an early caries lesion on an exposed tooth root surface, a dental composition that is capable of restoring the mineral density, if exists, is effective for restoring demineralization of the demineralized dentin. However, it is the current situation that there has been no proposal of a useful dental composition having such characteristics.

The inventions described in PTLs 1 and 2 intend to relieve dental hypersensitivity, and these patent literatures have no description nor suggestion about the restoration of the mineral density. PTL 1 does not disclose that a calcium ion and a hydrophilic solvent are blended in the anti-hyperesthesia agent. PTL 2 discloses that calcium is contained in the liquid B, but not in the liquid A coated in advance, and does not disclose that calcium is contained in the liquid A.

In view of the issues, a problem to be solved by the present invention is to provide a dental composition kit having a high mineral density restoration capability.

### Solution to Problem

As a result of earnest investigations by the present inventors for solving the problem, it has been newly found that the mineral density on a tooth surface can be restored with a dental composition kit including a liquid A and a liquid B, for example, a dental composition kit including a liquid A and a liquid B, the liquid A and the liquid B being coated on a tooth surface in this order, in which the liquid A contains a calcium ion, and the liquid B contains a compound having an acidic group and a zinc ion. Further investigations have been made based on the knowledge, and thus the present invention has been completed.

Specifically, the present invention encompasses the following inventions.

[1] A dental composition kit including a liquid A and a liquid B,

the liquid A containing a calcium ion (a1) and water (b1),
the liquid B containing a compound (c) having an acidic group represented by the following formula (I):

$$R^1\text{-}X_k \qquad \text{Formula (I)}$$

in which in the formula (I), $R^1$ represents an organic group having 40 or less carbon atoms having or not having at least one (meth)acryloyloxy group; X represents an acidic group; and k represents an integer of 1 or 2, in which in

the case where k represents 2, two acidic groups X are the same as or different from each other,
a zinc ion (d1), and water (b2), containing substantially no crosslinking polymerizable monomer.

[2] The dental composition kit according to the item [1], in which the liquid B further contains a hydrophilic solvent (e).
[3] The dental composition kit according to the item [1] or [2], in which the calcium ion (a1) is derived from at least one calcium compound (a) represented by the following formula (II):

$$(Ca^{2+})_c \cdot (Y^{n-})_d \qquad \text{Formula (II)}$$

in which in the formula (II), $Y^{n-}$ represents an anion as a counter ion of the calcium ion; n represents an integer of 1, 2, or 3; and c and d each represent an integer of 1, 2, or 3, in which c, d, and n have a relationship satisfying the equation 2c = nd.
[4] The dental composition kit according to any one of the items [1] to [3], in which the calcium ion (a1) is derived from at least one calcium compound (a) selected from the group consisting of $CaCl_2$, $CaBr_2$, $CaI_2$, $Ca(HCO_2)_2$, $Ca(HCO_3)_2$, and $Ca(CH_3COO)_2$.
[5] The dental composition kit according to any one of the items [1] to [4], in which the compound (c) having an acidic group is represented by the following formula (III):

$$R^1\text{-}((A^{m-})_a \cdot (H^+)_b)_k \qquad \text{Formula (III)}$$

in which in the formula (III), $R^1$ and k have the same meanings as defined in the formula (I); $A^{m-}$ represents an m-valent acid anionic group derived from the acidic group X; m represents an integer of 1, 2, or 3; and a and b each represent an integer of 1, 2, or 3, and have a relationship satisfying the equation am = b.
[6] The dental composition kit according to any one of the items [1] to [5], in which the compound (c) having an acidic group is a compound represented by the following formula (IV):

$$R^2\text{-}R^3\text{-O-P(=O)(-OH)}_2 \qquad \text{Formula (IV)}$$

in which in the formula (IV), $R^2$ represents a (meth)acryloyloxy group or hydrogen; and $R^3$ represents an alkylene group having 6 to 20 carbon atoms.
[7] The dental composition kit according to any one of the items [1] to [6], in which the zinc ion (d1) is derived from at least one zinc compound (d) selected from the group consisting of ZnO and a compound represented by the following formula (V):

$$(Zn^{2+})_h \cdot (P^{r-})_i \qquad \text{Formula (V)}$$

in which in the formula (V), $P^{r-}$ represents an anion as a counter ion of the zinc ion; r represents an integer of 1, 2, or 3; and h and i each represent an integer of 1, 2, or 3, in which h, i, and r have a relationship satisfying the equation 2h = ri.
[8] The dental composition kit according to any one of the items [1] to [7], in which the zinc ion (d1) is derived from at least one zinc compound (d) selected from the group consisting of ZnO, $ZnCl_2$, $Zn(OH)_2$, and $ZnF_2$.

## Advantageous Effects of Invention

The present invention provides a dental composition kit having a high mineral density restoration capability.

## Description of Embodiments

In the description herein, the upper limit values and the lower limit values of the numerical ranges (for example, the contents of the components, the values calculated from the components, and the physical properties) can be optionally combined.

### [Dental Composition Kit]

The dental composition kit according to an embodiment of the present invention is a dental composition kit including a liquid A and a liquid B,

the liquid A containing a calcium ion (a1) and water (b1),
the liquid B containing a compound (c) having an acidic group represented by the following formula (I):

$R^1$-$X_k$ Formula (I)

in which in the formula (I), $R^1$ represents an organic group having 40 or less carbon atoms having or not having at least one (meth)acryloyloxy group; X represents an acidic group; and k represents an integer of 1 or 2, in which in the case where k represents 2, two acidic groups X are the same as or different from each other,
a zinc ion (d1), and water (b2), containing substantially no crosslinking polymerizable monomer.

In the description herein, "(meth)acryloyloxy" is a generic term for methacryloyloxy and acryloyloxy. This rule is also applied to other similar terms.

According to the investigations by the present inventors, it has been found that the mineral density can be restored by coating the liquid A constituting the dental composition kit on a tooth surface, particularly an early caries lesion on an exposed tooth root surface, i.e., a demineralized portion of dentin losing minerals, and then further coating the liquid B thereon. The functional mechanism therefor can be estimated as follows, for example, while not limited thereto.

The liquid A containing a calcium ion is coated, and thereby the calcium ion penetrates into the layer of collagen fibers exposed through demineralization. The calcium ion penetrating into the layer of collagen fibers is deposited among the collagen fibers, and is considered to be the starting point of mineral recovery.

The layer formed with the liquid B is considered to have permeability to the mineral component contained in the saliva. Therefore, the liquid B is coated after coating the liquid A, and thereby the mineral is continuously fed to the demineralized portion via the layer formed with the liquid B, so as to increase the mineral component concentration in the demineralized portion. As a result, it is considered that the mineral is immobilized from the calcium ion fed from the liquid A as the starting point, and the mineral density of the demineralized dentin is recovered.

The order of coating on the tooth surface is preferably the liquid A and then the liquid B, but is not necessarily limited thereto, and the liquid B and the liquid A may be coated on the tooth surface in this order. In this case, it is considered that a part of the calcium ion derived from the liquid A reaches the layer of collagen fibers via the layer formed with the liquid B, and consequently the mineral density of the demineralized dentin is recovered through the same functional mechanism. In the case where the liquid B and the liquid A are coated in this order, the extent of the recovery of the mineral density tends to be smaller than the case where the liquid A and the liquid B are coated in this order, but the preservability of the layer formed with the liquid B is enhanced since the layer is formed directly on the tooth surface, which facilitates the stabilization of the feed of the mineral component from the saliva and the like.

<Liquid A>

The liquid A contains a calcium ion (a1) and water (b1). As described above, the liquid A is coated before the liquid B on the tooth surface, particularly the early caries lesion on the exposed tooth root surface, i.e., the demineralized portion of dentin losing minerals.

The liquid A containing a calcium ion (a1) coated allows the calcium ion (a1) to penetrate into the layer of collagen fibers exposed through demineralization, and the calcium ion (a1) is deposited among the collagen fibers, and is considered to be the starting point of mineral recovery.

(Calcium Ion (a1) and Calcium Compound (a))

The calcium ion (a1) is derived from a calcium compound (a), and the calcium compound (a) is dissolved in water (b1), so as to allow the calcium ion (a1) to be contained in the liquid A.

The calcium ion (a1) is derived, for example, from at least one calcium compound (a) represented by the following formula (II):

$(Ca^{2+})_c \cdot (Y^{n-})_d$ Formula (II)

in which in the formula (II), $Y^{n-}$ represents an anion as a counter ion of the calcium ion; n represents an integer of 1, 2, or 3; and c and d each represent an integer of 1, 2, or 3, in which c, d, and n have a relationship satisfying the equation 2c = nd.

The calcium ion (a1) is preferably derived from at least one calcium compound (a) selected from the group consisting of $CaCl_2$, $CaBr_2$, $CaI_2$, $Ca(HCO_2)_2$, $Ca(HCO_3)_2$, and $Ca(CH_3COO)_2$, more preferably derived from at least one calcium compound (a) selected from the group consisting of $CaCl_2$, $CaBr_2$, and $CaI_2$, and further preferably derived from $CaCl_2$, from the standpoint of the solubility in water (b1) and the safety in consideration of the use in the oral cavity.

One kind of the calcium compound (a) may be used alone for preparing the liquid A, or two or more kinds thereof may be used in combination. Therefore, one kind of the calcium ion (a1) may exist in the liquid A, or two or more kinds

thereof may exist.

The amount of the calcium compound (a) blended in the liquid A is preferably 10% by mass or more, more preferably 20% by mass or more, and further preferably 30% by mass or more, based on the mass of the water (b1) (100% by mass), from the standpoint of facilitating the sufficient penetration into the collagen fibers in the demineralized portion, and is preferably 200% by mass or less, more preferably 180% by mass or less, and further preferably 150% by mass or less, from the standpoint of preventing the excessive use of the calcium compound (a). In other words, the content of the calcium compound (a) is preferably 10 to 200% by mass based on the mass of the water (b1) as 100% by mass.

The content of the calcium ion (a1) in the liquid A is preferably 1.2% by mass or more, more preferably 2.3% by mass or more, and further preferably 3.1% by mass or more, and is preferably 10.7% by mass or less, more preferably 9.5% by mass or less, and further preferably 8.9% by mass or less, based on the total mass of the liquid A (100% by mass), from the same standpoints. In other words, the content of the calcium ion (a1) in the liquid A is preferably 1.2 to 10.7% by mass based on the total mass of the liquid A as 100% by mass.

The content of the calcium ion (a1) in the liquid A can be obtained, for example, through the measurement with an ICP emission spectrophotometer.

(Water (b1))

The water (b1) preferably does not contain harmful impurities, and is preferably distilled water or ion exchanged water. The content of the water (b1) in the liquid A is preferably 1 to 90% by mass, more preferably 5 to 85% by mass, and further preferably 10 to 80% by mass, based on the mass of the liquid A as 100% by mass.

(Amounts of Calcium Compound (a) and Water (b1) in Liquid A)

The total mass of the calcium compound (a) and the water (b1) in the liquid A is preferably 80 to 100% by mass, more preferably 90 to 100% by mass, and further preferably 95 to 100% by mass, based on the mass of the liquid A as 100% by mass, from the standpoint of facilitating the securement of the good permeability of calcium into the collagen fibers in the demineralized portion.

(Additional Component)

The liquid A may further contain one kind or two or more kinds of an additional component other than the calcium ion (a1) and the water (b1). Examples of the additional component include a pH modifier, a polymerization inhibitor, an ultraviolet ray absorbent, a thickener, a colorant (such as a dye and a pigment), a fluorescent agent, and a perfume. The liquid A may contain, as the additional component, a fluoride ion releasing substance, such as sodium fluoride, potassium fluoride, sodium monofluorophosphate, lithium fluoride, strontium fluoride, and ytterbium fluoride. The liquid A may contain a metal compound, such as silver oxide and aluminum hydroxide.

The content of the additional component is not particularly limited, may be such an amount that can provide the intended effects, and is preferably 0.01 to 1.5 parts by mass, more preferably 0.05 to 1.0 part by mass, and further preferably 0.07 to 0.5 part by mass, per 100 parts by mass of the liquid A, from the standpoint of facilitating the securement of the good permeability of calcium into the collagen fibers in the demineralized portion.

In the case where the additional component is an organic compound, the content thereof may be 5 parts by mass or less, and more preferably 3 parts by mass or less, per 100 parts by mass of the liquid A.

<Liquid B>

The liquid B contains a compound (c) having an acidic group represented by the following formula (I):

$$R^1\text{-}X_k \qquad \text{Formula (I)}$$

in which in the formula (I), $R^1$ represents an organic group having 40 or less carbon atoms having or not having at least one (meth)acryloyloxy group; X represents an acidic group; and k represents an integer of 1 or 2, in which in the case where k represents 2, two acidic groups X are the same as or different from each other, a zinc ion (d1), and water (b2), and contains substantially no crosslinking polymerizable monomer.

As described above, after coating the liquid A on the tooth surface, the liquid B is coated on the coating layer of the liquid A. According to the investigations by the present inventors, the liquid B is a material that has a function of repairing and preventing an early caries, and the liquid B coated on the early caries lesion protects and strengthens the weakened portion of dentin. On the other hand, the layer formed with the liquid B is considered to have permeability to the mineral component contained in the saliva as described above. Therefore, the mineral is continuously fed to the demineralized

portion via the layer formed with the liquid B, so as to increase the component concentration in the demineralized portion.

(Compound (c) having Acidic Group)

The compound (c) having an acidic group is a compound having an acidic group that allows an acidic composition formed by mixing with water (b2) and depending on necessity a hydrophilic solvent (e) to exhibit an acid etching effect and a primer treatment effect on the tooth substance, and is a component imparting a demineralization effect and a penetration effect.

The compound (c) having an acidic group is preferably a compound represented by the following formula (III):

$$R^1\text{-}((A^{m-})_a\cdot(H^+)_b)_k \qquad \text{Formula (III)}$$

in which in the formula (III), $R^1$ and k have the same meanings as defined in the formula (I); $A^{m-}$ represents an m-valent acid anionic group derived from the acidic group X; m represents an integer of 1, 2, or 3; and a and b each represent an integer of 1, 2, or 3, and have a relationship satisfying the equation am = b.

Examples of the compound (c) having an acidic group include a compound having at least one acidic group, such as a phosphoric acid group, a pyrophosphoric acid group, a thiophosphoric acid group, a phosphonic acid group, a sulfonic acid group, and a carboxylic acid group. Specific examples of the compound (c) having an acidic group are shown below.

Examples of the phosphoric acid group-containing compound include octyl dihydrogen phosphate, decyl dihydrogen phosphate, dodecyl dihydrogen phosphate (also known as dodecyl phosphate), tetradecyl dihydrogen phosphate, hexadecyl dihydrogen phosphate (also known as hexadecyl phosphate), octadecyl dihydrogen phosphate, 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl] hydrogen phosphate, bis[4-(meth)acryloyloxybutyl] hydrogen phosphate, bis[6-(meth)acryloyloxyhexyl] hydrogen phosphate, bis[8-(meth)acryloyloxyoctyl] hydrogen phosphate, bis[9-(meth)acryloyloxynonyl] hydrogen phosphate, bis[10-(meth)acryloyloxydecyl] hydrogen phosphate, 1,3-di(meth)acryloyloxypropyl dihydrogen phosphate, 2-(meth)acryloyloxyethylphenyl hydrogen phosphate, 2-(meth)acryloyloxyethyl-2-bromoethyl hydrogen phosphate, and bis[2-(meth)acryloyloxy-(1-hydroxymethyl)ethyl] hydrogen phosphate.

Examples of the pyrophosphoric acid group-containing compound include bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, and bis[10-(meth)acryloyloxydecyl] pyrophosphate.

Examples of the thiophosphoric acid group-containing compound include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, and 20-(meth)acryloyloxyicosyl dihydrogen thiophosphate.

Examples of the phosphonic acid group-containing compound include 2-(meth)acryloyloxyethyl phenylphosphonate, 5-(meth)acryloyloxypentyl 3-phosphonopropionate, 6-(meth)acryloyloxyhexyl 3-phosphonopropionate, 10-(meth)acryloyloxydecyl 3-phosphonopropionate, 6-(meth)acryloyloxyhexyl phosphonoacetate, and 10-(meth)acryloyloxydecyl phosphonoacetate.

Examples of the sulfonic acid group-containing compound include 2-(meth)acrylamido-2-methylpropanesulfonic acid, styrenesulfonic acid, and 2-sulfoethyl (meth)acrylate.

Examples of the carboxylic acid group-containing compound include a compound having one carboxy group in the molecule and a compound having multiple carboxy groups in the molecule.

Examples of the compound having one carboxy group in the molecule include (meth)acrylic acid, N-(meth)acryloylglycine, N-(meth)acryloylaspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxyethyl hydrogen succinate, 2-(meth)acryloyloxyethyl hydrogen phthalate, and 2-(meth)acryloyloxyethyl hydrogen maleate.

Examples of the compound having multiple carboxy groups in the molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxybutyl trimellitate, 4-(meth)acryloyloxyhexyl trimellitate, 4-(meth)acryloyloxydecyl trimellitate, and 2-(meth)acryloyloxyethyl-3'-(meth)acryloyloxy-2'-(3,4-dicarboxybenzoyloxy)propyl succinate.

The compound (c) having an acidic group is more preferably a compound represented by the following formula (IV):

$$R^2\text{-}R^3\text{-O-P(=O)(-OH)}_2 \qquad \text{Formula (IV)}$$

in which in the formula (IV), $R^2$ represents a (meth)acryloyloxy group or hydrogen; and $R^3$ represents an alkylene group having 6 to 20 carbon atoms.

In the compound (c) having an acidic group, a phosphoric acid group-containing or pyrophosphoric acid group-containing compound is preferred since the compound exhibits further excellent deposition formation on the tooth substance, and a phosphoric acid group-containing compound is particularly preferred. Especially, in view of the high tooth substance demineralization and the high deposition formation exhibited thereby, a divalent phosphoric acid group-containing compound having an alkyl group having 6 to 20 carbon atoms or an alkylene group as a main chain in the molecule is more preferred, and a divalent phosphoric acid group-containing compound having an alkyl group having 8 to 12 carbon atoms or an alkylene group as a main chain, such as dodecyl dihydrogen phosphate and 10-methacryloyloxydecyl dihydrogen phosphate, is most preferred.

One kind of the compound (c) having an acidic group may be used alone, or two or more kinds thereof may be used in combination.

The content of the compound (c) having an acidic group is preferably 10% by mass or more, more preferably 15% by mass or more, and further preferably 20% by mass or more, based on the total mass of the compound (c) having an acidic group, the zinc compound (d) described later, and water (b2) in the liquid B as 100% by mass, from the standpoint of facilitating the securement of the effects of the present invention. The content thereof is preferably 70% by mass or less, and more preferably 60% by mass or less. In other words, the content of the compound (c) having an acidic group is preferably 10 to 70% by mass based on the total mass of the compound (c) having an acidic group, the zinc compound (d), and water (b2) as 100% by mass.

In the case where the liquid B contains a hydrophilic solvent (e), the content of the compound (c) having an acidic group is preferably 5% by mass or more, more preferably 10% by mass or more, and further preferably 15% by mass or more, based on the total mass of the compound (c) having an acidic group, the zinc compound (d), water (b2), and the hydrophilic solvent (e) in the liquid B as 100% by mass, from the standpoint of facilitating the securement of the effects of the present invention. The content thereof is preferably 50% by mass or less, and more preferably 40% by mass or less. In other words, the content of the compound (c) having an acidic group is preferably 5 to 50% by mass based on the total mass of the compound (c) having an acidic group, the zinc compound (d), water (b2), and the hydrophilic solvent (e) as 100% by mass.

(Zinc Ion (d1))

The zinc ion (d1) is derived from a zinc compound (d), and the zinc compound (d) is dissolved through the compound (c) having an acidic group, so as to allow the zinc ion (d1) to be contained in the liquid B.

The zinc ion (d1) is preferably derived from at least one zinc compound (d) selected from the group consisting of ZnO and a compound represented by the following formula (V):

$$(Zn^{2+})_h \cdot (P^{r-})_i \qquad \text{Formula (V)}$$

in which in the formula (V), $P^{r-}$ represents an anion as a counter ion of the zinc ion; r represents an integer of 1, 2, or 3; and h and i each represent an integer of 1, 2, or 3, in which h, i, and r have a relationship satisfying the equation 2h = ri.

The zinc ion (d1) is preferably derived from at least one zinc compound (d) selected from the group consisting of ZnO, $ZnCl_2$, $Zn(OH)_2$, and $ZnF_2$, and more preferably derived from ZnO.

One kind of the zinc compound (d) may be used alone for preparing the liquid B, or two or more kinds thereof may be used in combination. Therefore, one kind of the zinc ion (d1) may exist in the liquid B, or two or more kinds thereof may exist.

The content of the zinc compound (d) blended in the liquid B is preferably 0.5% by mass or more, more preferably 1.0% by mass or more, and further preferably 1.5% by mass or more, based on the total mass of the compound (c) having an acidic group and the water (b2) (100% by mass), from the standpoint of facilitating the favorable formation of the deposit for strengthening the tooth substance, and is preferably 15% by mass or less, more preferably 12% by mass or less, and further preferably 10% by mass or less, from the standpoint of suppressing the excessive progress of neutralization of the

acidic group of the compound (c) having an acidic group, and securing the favorable demineralization function and penetration function. In other words, the content of the zinc compound (d) in the liquid B is preferably 0.5 to 15% by mass based on the total mass of the compound (c) having an acidic group and the water (b2) as 100% by mass.

In the case where the liquid B contains a hydrophilic solvent (e), the content of the zinc compound (d) blended in the liquid B is preferably 0.3% by mass or more, more preferably 0.6% by mass or more, and further preferably 0.8% by mass or more, and is preferably 10% by mass or less, more preferably 7.0% by mass or less, and further preferably 5.0% by mass or less, based on the total mass of the compound (c) having an acidic group, the water (b2), and the hydrophilic solvent (e) (100% by mass), from the same standpoints. In other words, the content of the zinc compound (d) in the liquid B is preferably 0.3 to 10% by mass based on the total mass of the compound (c) having an acidic group, the water (b2), and the hydrophilic solvent (e) as 100% by mass.

The content of the zinc ion (d1) in the liquid B is preferably 0.2% by mass or more, more preferably 0.4% by mass or more, and further preferably 0.7% by mass or more, and is preferably 12% by mass or less, more preferably 10% by mass or less, and further preferably 8.0% by mass or less, based on the total mass of the compound (c) having an acidic group and the water (b2) as 100% by mass, from the same standpoints. In other words, the content of the zinc ion (d1) in the liquid B is preferably 0.2 to 12% by mass based on the total mass of the compound (c) having an acidic group and the water (b2) as 100% by mass.

In the case where the liquid B contains a hydrophilic solvent (e), the content of the zinc ion (d1) in the liquid B is preferably 0.1% by mass or more, more preferably 0.2% by mass or more, and further preferably 0.3% by mass or more, and is preferably 8.0% by mass or less, more preferably 5.6% by mass or less, and further preferably 4.0% by mass or less, based on the total mass of the compound (c) having an acidic group, the water (b2), and the hydrophilic solvent (e) as 100% by mass, from the same standpoints. In other words, the content of the zinc ion (d1) in the liquid B is preferably 0.1 to 8.0% by mass based on the total mass of the compound (c) having an acidic group, the water (b2), and the hydrophilic solvent (e) as 100% by mass.

The content of the zinc ion (d1) in the liquid B can be calculated from the amount of the zinc compound (d) blended, and the content of the zinc ion (d1) in the liquid B can also be obtained through the measurement with an ICP emission spectrophotometer.

(Water (b2))

The water (b2) preferably does not contain harmful impurities, and is preferably distilled water or ion exchanged water. The content of the water (b2) in the liquid B is preferably 1 to 90% by mass, more preferably 5 to 80% by mass, and further preferably 10 to 70% by mass, based on the total mass of the compound (c) having an acidic group, the zinc compound (d), and the water (b2) as 100% by mass.

In the case where the liquid B contains a hydrophilic solvent (e), the content of the water (b2) in the liquid B is preferably 1 to 50% by mass, more preferably 5 to 40% by mass, and further preferably 10 to 30% by mass, based on the total mass of the compound (c) having an acidic group, the zinc compound (d), the water (b2), and the hydrophilic solvent (e) as 100% by mass.

(Amounts of Compound (c) having Acidic Group, Zinc Compound (d), and Water (b2) in Liquid B)

The total mass of the compound (c) having an acidic group, the zinc compound (d), and the water (b2) in the liquid B is preferably 10 to 60% by mass, more preferably 20 to 55% by mass, and further preferably 30 to 50% by mass, based on the mass of the liquid B as 100% by mass, from the standpoint of the favorable formation of the deposit for strengthening the tooth substance.

In the case where the liquid B contains a hydrophilic solvent (e), the total mass of the compound (c) having an acidic group, the zinc compound (d), the water (b2), and the hydrophilic solvent (e) in the liquid B is preferably 85 to 100% by mass, more preferably 90 to 100% by mass, and further preferably 93 to 100% by mass, based on the mass of the liquid B as 100% by mass, from the standpoint of the favorable formation of the deposit for strengthening the tooth substance, and the favorable coatability to the tooth surface.

(Hydrophilic Solvent (e))

The liquid B may further contain a hydrophilic solvent (e) as an optional component. The hydrophilic solvent (e) means an organic compound having a solubility in water at 25°C of 10% by mass or more, which preferably has the solubility that is 30% by mass or more, and is more preferably capable of being dissolved in water at 25°C in an arbitrary proportion.

The hydrophilic solvent (e) may be a single compound, or may be a mixture of multiple compounds.

Examples of the hydrophilic solvent (e) include ethanol, 2-propanol, 2-methyl-2-propanol, acetone, tetrahydrofuran, 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, polyethylene glycol di(meth)acrylate (having a number of oxyethylene groups of 9 or more), N,N-dimethylaminoethyl methacrylate, N-methylol (meth)acrylamide, N-hydroxyethyl (meth)acrylamide, N,N-bis(2-hydroxyethyl) (meth)acrylamide, N-methoxymethyl (meth)acrylamide, N-ethoxymethyl (meth)acrylamide, diacetone (meth)acrylamide, 4-(meth) acryloylmorpholine, N-trihydroxymethyl-N-methyl (meth)acrylamide, N-[3-(dimethylamino)propyl]acrylamide, and a (meth)acrylamide represented by the following formula (VI):

[Chem 1]

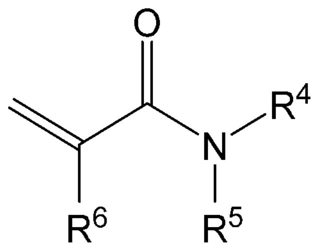

(VI)

in which in the formula (VI), $R^4$ and $R^5$ each independently represent an alkyl group having 1 to 3 carbon atoms (such as a methyl group, an ethyl group, a n-propyl group, or an isopropyl group), which may have a substituent (such as a hydroxy group); and $R^6$ represents a hydrogen atom or a methyl group.

In the hydrophilic solvent (e), ethanol, 2-propanol, 2-methyl-2-propanol, acetone, tetrahydrofuran, 2-hydroxyethyl (meth)acrylate, 2,3-dihydroxypropyl (meth)acrylate, N,N-dimethylaminoethyl methacrylate, diacetone (meth) acrylamide, and a monofunctional (meth)acrylamide-based monomer are preferred, and ethanol, 2-propanol, 2-hydroxyethyl (meth)acrylate, N,N-dimethylaminoethyl methacrylate, and a (meth)acrylamide-based monomer represented by the formula (VI) are more preferred. One kind of the hydrophilic solvent (e) may be blended alone, or two or more kinds thereof may be used in combination.

The hydrophilic solvent (e) functions as a cosolvent of the compound (c) having an acidic group and the water (b2). The content of the hydrophilic solvent (e) in the liquid B is preferably 1 to 90% by mass, more preferably 5 to 80% by mass, and further preferably 10 to 70% by mass, based on the total mass of the compound (c) having an acidic group, the zinc compound (d), the water (b2), and the hydrophilic solvent (e) as 100% by mass.

<Organic Compound other than Compound (c) having Acidic Group and Hydrophilic Solvent (e)>

The liquid B contains substantially no crosslinking polymerizable monomer, which is an organic compound other than the compound (c) having an acidic group and the hydrophilic solvent (e). The expression "containing substantially no" herein specifically means that the crosslinking polymerizable monomer is 5 parts by mass or less, and preferably 3 parts by mass or less, per 100 parts by mass of the liquid B.

Examples of the crosslinking polymerizable monomer include a hydrophobic crosslinking polymerizable monomer having no acidic group, for example, an aromatic bifunctional monomer, an aliphatic bifunctional monomer, and a trifunctional or higher functional monomer.

The liquid B according to the embodiment of the present invention contains substantially no crosslinking polymerizable monomer, and thereby the function of forming a composite of the compound (c) having an acidic group, the zinc ion (d1), and the inorganic component of the demineralized tooth substance is prevented from being impaired to accelerate the formation of the deposition with these materials.

Examples of the aromatic bifunctional monomer include 2,2-bis((meth)acryloyloxyphenyl)propane, 2,2-bis [4-(3-(meth)acryloyloxy-2-hydroxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane.

Examples of the aliphatic bifunctional monomer include glycerol di(meth)acrylate, ethylene glycol di(meth) acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol

di(meth)acrylate, 1,6-hexanediol di(meth)acrylate, 1,10-decanediol di(meth)acrylate, 1,2-bis(3-methacryloyloxy-2-hydroxypropoxy)ethane, 2,2,4-trimethylhexamethylene bis(2-carbamoyloxyethyl) di(meth)acrylate, N-methacryloyloxyethyl acrylamide, N-methacryloyloxypropyl acrylamide, N-methacryloyloxybutyl acrylamide, N-(1-ethyl-(2-methacryloyloxy)ethyl)acrylamide, and N-(2-(2-methacryloyloxyethoxy)ethyl)acrylamide.

Examples of the trifunctional or higher functional monomer include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy) propane-1,3-diol] tetra(meth)acrylate, and 1,7-diacryloyloxy-2,2,6,6-tetra(meth)acryloyloxymethyl-4-oxyheptane.

The liquid B preferably contains substantially no organic compound other than the compound (c) having an acidic group and the hydrophilic solvent (e), including the crosslinking polymerizable monomer. The expression "containing substantially no" herein specifically means that the organic compound other than the compound (c) having an acidic group and the hydrophilic solvent (e) is 5 parts by mass or less, and preferably 3 parts by mass or less, per 100 parts by mass of the liquid B.

Examples of the organic compound other than the compound (c) having an acidic group and the hydrophilic solvent (e) that is not the crosslinking polymerizable monomer include:

a photopolymerization initiator, such as a (bis)acylphosphine oxide compound, a thioxanthone compound or a quaternary ammonium salt of a thioxanthone compound, a ketal compound, an $\alpha$-diketone compound, a coumarin compound, an anthraquinone compound, a benzoin alkyl ether compound, and an $\alpha$-aminoketone-based compound;
a polymerization accelerator, such as an aldehyde compound, a thiol compound, and an aminobenzoate ester compound;
an antimicrobial substance, such as cetylpyridinium chloride, benzalkonium chloride, (meth)acryloyloxydodecylpyridinium bromide, (meth)acryloyloxyhexadecylpyridinium chloride, (meth)acryloyloxydecylammonium chloride, triclosan, and 12-methacryloyloxydodecylpyridinium bromide; and
a monofunctional polymerizable monomer, such as 4-hydroxybutyl (meth)acrylate, 6-hydroxyhexyl (meth)acrylate, 8-hydroxyoctyl (meth)acrylate, 10-hydroxydecyl (meth)acrylate, and octafluoropentyl (meth)acrylate.

Examples of the (bis)acylphosphine oxide compound used as the photopolymerization initiator include 2,4,6-trimethylbenzoyl diphenylphosphine oxide, 2,4,6-trimethylbenzoyl methoxyphenylphosphine oxide, bis(2,4,6-trimethylbenzoyl)phenylphosphine oxide, and 2,4,6-trimethylbenzoyl phenylphosphine oxide sodium salt.

Examples of the $\alpha$-diketone compound used as the photopolymerization initiator include diacetyl, benzil, camphorquinone, 2,3-pentanedione, 2,3-octanedione, 9,10-phenanthrenequinone, 4,4'-oxybenzil and acenaphthenequinone.

Specific examples of the aldehyde compound used as the polymerization accelerator include terephthalaldehyde and a benzaldehyde derivative. Examples of the benzaldehyde derivative include dimethylaminobenzaldehyde, p-methoxybenzaldehyde, p-ethoxybenzaldehyde, and p-n-octyloxybenzaldehyde.

Specific examples of the thiol compound used as the polymerization accelerator include 2-mercaptobenzoxazole, decanethiol, 3-mercaptopropyltrimethoxysilane, and thiobenzoic acid.

Specific examples of the thiol compound used as the polymerization accelerator include aminobenzoate ester compound include ethyl 4-(N,N-dimethylamino)benzoate, methyl 4-(N,N-dimethylamino)benzoate, n-butoxyethyl 4-N,N-(dimethylamino)benzoate, 2-[(meth)acryloyloxy]ethyl 4-(N,N-dimethylamino)benzoate, 4-(N,N-dimethylamino)benzophenone, and n-butyl 4-(N,N-dimethylamino)benzoate.

<Additional Component>

The liquid B may further contain one kind or two or more kinds of an additional component. Examples of the additional component include a pH modifier, a polymerization inhibitor, an ultraviolet ray absorbent, a thickener, a colorant (such as a dye and a pigment), a fluorescent agent, and a perfume. The liquid B may contain, as the additional component, a fluoride ion releasing substance, such as sodium fluoride, potassium fluoride, sodium monofluorophosphate, lithium fluoride, strontium fluoride, and ytterbium fluoride. The liquid B may contain a metal compound, such as calcium hydroxide, calcium chloride, silver oxide, and aluminum hydroxide.

The content of the additional component is not particularly limited, may be such an amount that can provide the intended effects, and is preferably 0.01 to 1 part by mass, more preferably 0.05 to 0.5 part by mass, and further preferably 0.07 to 0.3 part by mass, per 100 parts by mass of the liquid B, from the standpoint of facilitating the securement of the permeability of the mineral component.

In the case where the additional component is an organic compound, the content thereof may be 8 parts by mass or less, and more preferably 5 parts by mass or less, per 100 parts by mass of the liquid B.

[Characteristics of Dental Composition Kit]

The dental composition kit according to the embodiment of the present invention exhibits a high mineral density restoration capability by coating the liquid A and the liquid B on the tooth surface, particularly by coating the liquid A and the liquid B in this order on the tooth surface, as described above.

The mineral density restoration capability of the dental composition kit is calculated in such a manner that the liquid A and the liquid B are coated on a sample of a demineralized bovine tooth, which is immersed in artificial saliva under the prescribed condition, and the tooth surface of the sample is measured with contact microradiography (CMR) to provide an image, the brightness values of which are analyzed and integrated at each of the prescribed depths to provide a value with respect to a sample of a bovine tooth having no liquid A nor liquid B coated thereon, and specifically is measured and calculated by the procedure described in the examples.

The mineral density restoration capability of the dental composition kit according to the embodiment of the present invention is preferably 17% or more, more preferably 18% or more, further preferably 19% or more, and still further preferably 20% or more. The upper limit thereof is not particularly limited, and is, for example, 35% from the standpoint of the manufacturability of the dental composition kit. In other words, the mineral density restoration capability of the dental composition kit according to the embodiment of the present invention is preferably 17 to 35%.

[Production Method of Liquid A and Liquid B]

As a production method of the liquid A, the liquid A can be obtained by mixing the calcium compound (a) and the water (b1) to provide a state where the calcium compound (a) as a solid component is dissolved in the water (b1). In the case where the liquid A contains the additional component, the additional component may be mixed at any timing.

As a production method of the liquid B, the compound (c) having an acidic group, the zinc compound (d), the water (b2), and depending on necessity the hydrophilic solvent (e) may be mixed in any order, and a state where the zinc compound (d) as a solid component is dissolved in the liquid mixed composition containing the compound (c) having an acidic group, the water (b2), and the hydrophilic solvent (e) is provided finally, resulting in the liquid B. In the case where the liquid B contains the additional component, the additional component may be mixed at any timing.

[Use Method of Dental Composition Kit]

The liquid A and the liquid B each may be coated on the tooth substance with a swab, a sponge piece, a small brush, an applicator brush, or the like. Depending on necessity after coating, the volatile component is dried through air blowing with a three-way syringe or the like, so as to complete the treatment. It is preferred that the liquid A is coated and then dried, and then the liquid B is coated.

The coating of the liquid A and the liquid B on the tooth substance is a simple operative procedure, and therefore even in the case where a large number of teeth are treated, the stresses experienced by both the practitioner and the patient are small.

The present invention encompasses embodiments including the aforementioned configurations combined variously within the scope of the technical concept of the present invention, as long as the effects of the present invention are exhibited.

Examples

The dental composition kit according to the present invention will be specifically described with reference to examples below, but the dental composition kit according to the present invention is not limited to the examples.

The components used in Examples and Comparative Examples were as follows.

[Compound (c) having Acidic Group]

MHP: 6-methacryloyloxyhexyl dihydrogen phosphate
MDP: 10-methacryloyloxydecyl dihydrogen phosphate
M12P: 12-methacryloyloxydodecyl dihydrogen phosphate
4-MET: 4-methacryloyloxyethyl trimellitate

[Hydrophilic Solvent (e)]

HEMA: 2-hydroxyethyl methacrylate
DEAA: N,N-diethylacrylamide (compound represented by the formula (VI))
DMAEMA: N,N-dimethylaminoethyl methacrylate

[Organic Compound other than Compound (c) having Acidic Group and Hydrophilic Solvent (e)]

CPC: cetylpyridinium chloride
MDPB: 12-methacryloyloxydodecylpyridinium bromide
Bis-GMA: 2,2-bis[4-(3-methacryloyloxy-2-hydroxypropoxy)phenyl]propane
CQ: DL-camphorquinone
EDMB: ethyl 4-(N,N-dimethylamino)benzoate

[Preparation and Evaluation of Dental Composition Kit]

<Examples 1 to 11 and Comparative Examples 1 to 4>

The materials were mixed according to the formulation shown in Table 1 to prepare the liquid A and the liquid B of each of Examples and Comparative Examples, which were designated as dental composition kits. Samples were prepared from the dental composition kits in the following manner, and the samples were measured and evaluated in the following manner.

In Comparative Example 1, only the liquid B was used, but no liquid A was used. In Example 11, the liquid B in Table 1 was used in advance as the liquid A in the section "(4) Production of Test Piece", and the liquid A in Table 1 was then used as the liquid B in the section "(4) Production of Test Piece".

<Evaluation of Mineral Density Restoration Capability>

The characteristic feature capable of accelerating the remineralization of the demineralized portion of dentin was evaluated in the following manner as the mineral density restoration capability.

(1) Production of Demineralizing Liquid

A 50 mM aqueous solution of acetic acid (available from Tokyo Kasei Kogyo Co., Ltd.) and a 50 mM aqueous solution of sodium acetate trihydride (available from Fujifilm Wako Pure Chemical Corporation) were mixed and regulated to pH 4.5.

(2) Preparation of Artificial Saliva

87.6 mg of sodium chloride (available from Fujifilm Wako Pure Chemical Corporation), 122 mg of potassium dihydrogen phosphate (available from Fujifilm Wako Pure Chemical Corporation), 166 mg of calcium chloride (available from Fujifilm Wako Pure Chemical Corporation), and 477 mg of 2-[4-(2-hydroxyethyl)-1-piperazinyl]ethanesulfonic acid (HEPES) (available from Dojindo Laboratories Co., Ltd.) were dissolved in approximately 500 mL of water and regulated to pH 7.0 with a saturated aqueous solution of NaOH (available from Fujifilm Wako Pure Chemical Corporation), which was then made up to 1.0 L.

(3) Reagents used for producing Test Piece

The following compounds were used for producing a test piece.

Ethanol: available from Kanto Chemical Co., Inc.
Propylene oxide: available from Fujifilm Wako Pure Chemical Corporation
Epoxy resin base resin: EpoxiCure 2 Base Resin (available from Buehler Ltd.)
Curing agent: EpoxiCure 2 Curing Agent (available from Buehler Ltd.)

(4) Production of Test Piece

A test piece was produced in the following manner by using a cervical portion of a bovine tooth.

The surface of a cervical portion of a bovine tooth was polished with #80 polishing paper to expose dentin, and then further polished with #1000 polishing paper, followed by subjecting to an ultrasonic treatment for 5 minutes. A part of the surface of the test piece of the cervical portion of the bovine tooth subjected to the treatment above was designated as a test window of approximately 5 mm × 2 mm, and the test piece of the cervical portion of the bovine tooth was bisected with a diamond cutter (Isomet 1000, available from Buehler Ltd.) so that the region corresponding to the test window became bilaterally symmetric with the divided surface as the center.

In both the bisected test pieces, the portion other than the test window was coated with a manicure to produce two test pieces each having a test window of 5 mm × 1 mm. The test pieces were immersed in the demineralizing liquid, and demineralized at 37°C for 2 weeks. From the start of immersing in the demineralizing liquid, the demineralizing liquid was renewed once every three days.

The manicure on the back surface of each of the test pieces was peeled off, and then a manicure was coated on the test window (test window A) of one of the test pieces, followed by drying.

The test window (test window B) of the other one of the test pieces was immersed in 4 μL of the artificial saliva for 1 hour, and the dried by air blowing, and the sample (i.e., the dental composition kit of each of Examples and Comparative Example) was coated thereon. The dental composition kit was coated on the test window B in the following manner. First, the liquid A was coated on the test window B, allowed to stand for 10 seconds, and then dried by air blowing, and subsequently, the liquid B was coated thereon, allowed to stand for 10 seconds, and then dried by air blowing. Both the test pieces were immersed in the artificial saliva, and stored at 37°C for 14 days. The artificial saliva was renewed 10 times during 14 days from the start of immersing.

The manicure was peeled off from the resulting test pieces, which were then immersed in 70% by volume ethanol, 80% by volume ethanol, 90% by volume ethanol, 99% by volume ethanol, and 100% by volume ethanol each for 10 minutes, and then immersed in 100% by volume ethanol for one day. The test pieces were immersed in a mixture of propylene oxide and ethanol (1/1 by volume) and propylene oxide each for 10 minutes, and then immersed in propylene oxide for one day. The test pieces were immersed in a mixed liquid of an epoxy resin base resin and propylene oxide (1/1 by volume), a mixed liquid of an epoxy resin base resin and propylene oxide (4/1 by volume), and an epoxy resin base resin each for 2 hours, then immersed in a mixed liquid of an epoxy resin base resin and propylene oxide (4/1 by volume), and stored while immersed at 37°C for one day for curing. The test pieces as cured products each were cut into a thickness of approximately 1.2 mm perpendicular to the long edge of the test window with a diamond cutter (Isomet 1000, available from Buehler Ltd.), and the thickness of the test piece was regulated to 0.100 mm to 0.150 mm with lapping films (#1200, #3000, and #8000, available from 3M Japan Co., Ltd.).

(5) Acquisition of CMR Image

For each of the resulting test pieces, the entire test piece was imaged with a soft X-ray inspection equipment ("CMR-2", trade name, available from Softex Co., Ltd.) and a glass dry plate (High Precision Photo Plate HPP-SN2 2×2, available from Konica Minolta Co., Ltd.) at a tube voltage of 10 to 15 kV, a tube current of 2.0 mA, and an irradiation time of 10 minutes, for acquiring an image of contact microradiography (CMR) (CMR image). The imaged test piece (dry plate) was immersed in a developer solution (Medical X-ray Liquid Developer "Hi-RENDOL", available from Fujifilm Corporation) for 5 minutes, rinsed with distilled water for 1 minute, immersed in a fixing solution (Fixing Agent "Hi-RENFIX", available from Fujifilm Corporation) for 5 minutes, and rinsed with distilled water for 30 seconds.

(6) Calculation of Mineral Density Restoration Capability through Analysis of CMR Image

The resulting CMR image was imaged with a microscope (Nikon DS-Ri1, available from Nikon Corporation), and the brightness values was measured from the surface of the test window in the depth direction of the test window, with the brightness value at a depth of 700 μm of the test window designated as the standard, while setting the measurement interval of 5 μm in the depth range of 0 μm to 700 μm, with an image analysis software (Image J, open source, public domain), from which the mineral density restoration capability (%) was obtained according to the following equation 1.

$$\text{Mineral density restoration capability (\%)} = \{[(DT_0\text{-}DC_0)+(DT_5\text{-}DC_5)+(DT_{10}\text{-}DC_{10})+...+(DT_{700}\text{-}DC_{700})]/[(1\text{-}DC_0)+(1\text{-}DC_5)+(1\text{-}DC_{10})+...+(1\text{-}DC_{700})]\}\times 100 \quad \text{(equation 1)}$$

The symbols in the equation 1 have the following meanings. In the following description, "control" means the case that is not treated with the sample (i.e., the dental composition kit of each of Examples and Comparative Examples).

$DC_x$: control mineral density at depth of x μm
$BC_x$: control brightness value at depth of x μm
$DT_x$: mineral density of treated surface at depth of x μm
$BT_x$: brightness value of treated surface at depth of x μm

The values of $DC_x$ and $DT_x$ were calculated according to the following equations.

$$DC_x = BC_x/BC_{700} \qquad \text{(equation 2)}$$

$$DT_x = BT_x/BT_{700} \qquad \text{(equation 3)}$$

Three test pieces were produced for each of Examples and Comparative Examples, and the test pieces each were measured for the mineral density restoration capability by the aforementioned procedure, the arithmetic average value of which was obtained and designated as the mineral density restoration capability of each of Examples and Comparative Examples. The measurement results of Examples and Comparative Examples are shown in Table 1 along with the compositions of the dental composition kits.

[Table 1]

Table 1

| | Kind of component | Name of component | Unit | Example | | | | | | | |
|---|---|---|---|---|---|---|---|---|---|---|---|
| | | | | 1 | 2 | 3 | 4 | 5 | 6 | 7 | 8 |
| Liquid A | (a) | $CaCl_2$ | part by mass | 42.9 | 42.9 | 42.9 | 42.9 | 42.8 | 42.8 | - | - |
| | | $CaBr_2$ | part by mass | - | - | - | - | - | - | 57.8 | - |
| | | $CaI_2$ | part by mass | - | - | - | - | - | - | - | 39.6 |
| | (b1) | Water | part by mass | 57.1 | 57.1 | 57.1 | 57.1 | 57.1 | 57.1 | 41.2 | 60.2 |
| | Additional component | NaF | part by mass | - | - | - | - | 0.1 | - | 0.1 | - |
| | | $AlCl_3$ | part by mass | - | - | - | - | - | 0.1 | - | - |
| | | $AlK(SO_4)_2$ | part by mass | - | - | - | - | - | - | 0.9 | - |
| | | $CuCl_2$ | part by mass | - | - | - | - | - | - | - | 0.2 |

(continued)

| | Kind of component | Name of component | Unit | Example | | | | | | | |
|---|---|---|---|---|---|---|---|---|---|---|---|
| | | | | 1 | 2 | 3 | 4 | 5 | 6 | 7 | 8 |
| Liquid B | (c) | Dodecyl phosphate | part by mass | 30.0 | - | - | - | - | - | - | - |
| | | Hexadecyl phosphate | part by mass | - | 30.0 | - | - | - | - | 30.0 | 25.0 |
| | | MHP | part by mass | - | - | 30.0 | - | - | - | - | - |
| | | MDP | part by mass | - | - | - | 30.0 | - | 30.0 | - | - |
| | | M12P | part by mass | - | - | - | - | 30.0 | - | - | - |
| | | 4-MET | part by mass | - | - | - | - | - | - | - | - |
| | (d) | ZnO | part by mass | 0.4 | 0.4 | 0.4 | 0.4 | 0.4 | 0.4 | 0.4 | - |
| | | $ZnCl_2$ | part by mass | 2.5 | 2.5 | 2.5 | 2.5 | 2.5 | 2.5 | 2.5 | 2.5 |
| | | $Zn(OH)_2$ | part by mass | - | - | - | - | - | - | - | 0.4 |
| | (b2) | Water | part by mass | 12.0 | 12.0 | 12.0 | 12.0 | 12.0 | 12.0 | 12.0 | 10.0 |
| | (e) | Ethanol | part by mass | 53.0 | 58.0 | 58.0 | 53.0 | - | - | - | 55.0 |
| | | Isopropanol | part by mass | - | - | - | - | 58.0 | - | - | - |
| | | HEMA | part by mass | - | - | - | - | - | 53.0 | - | - |
| | | DEAA | part by mass | - | - | - | - | - | - | 51.0 | - |
| | | DMAEMA | part by mass | 5.0 | - | - | 5.0 | - | 5.0 | 7.0 | 10.0 |

(continued)

| | Kind of component | Name of component | Unit | Example | | | | | | | |
|---|---|---|---|---|---|---|---|---|---|---|---|
| | | | | 1 | 2 | 3 | 4 | 5 | 6 | 7 | 8 |
| | Organic compound other than (c) and (e) | CPC | part by mass | 5.2 | - | - | - | - | - | - | - |
| | | MDPB | part by mass | - | - | - | 5.0 | 5.0 | - | - | - |
| | | Bis-GMA | part by mass | - | - | - | - | - | - | - | - |
| | | CQ | part by mass | - | - | - | - | - | - | - | - |
| | | EDMB | part by mass | - | - | - | - | - | - | - | - |
| | Additional component | NaF | part by mass | - | - | - | - | 0.1 | 0.1 | 0.1 | - |
| | | KF | part by mass | 0.1 | 0.1 | 0.1 | 0.1 | - | - | - | 0.1 |
| Mineral density restoration capability | | | % | 26.9 | 27.5 | 27.6 | 28.8 | 26.7 | 27.1 | 25.8 | 23.9 |

Table 1 (continued)

| | Kind of component | Name of component | Unit | Example | | | Comparative Example | | | |
|---|---|---|---|---|---|---|---|---|---|---|
| | | | | 9 | 10 | 11 | 1 | 2 | 3 | 4 |
| Liquid A | (a) | $CaCl_2$ | part by mass | 40.0 | 30.0 | 42.9 | none | 42.9 | 42.9 | 42.9 |
| | | $CaBr_2$ | part by mass | - | - | - | | - | - | - |
| | | $CaI_2$ | part by mass | - | - | - | | - | - | - |
| | (b1) | Water | part by mass | 60.0 | 70.0 | 57.1 | | 57.1 | 57.1 | 57.1 |
| | Additional component | NaF | part by mass | - | - | - | | - | - | - |
| | | $AlCl_3$ | part by mass | - | - | - | | - | - | - |
| | | $AlK(SO_4)_2$ | part by mass | - | - | - | | - | - | - |
| | | $CuCl_2$ | part by mass | - | - | - | | - | - | - |

(continued)

| | Kind of component | Name of component | Unit | Example | | | Comparative Example | | | |
|---|---|---|---|---|---|---|---|---|---|---|
| | | | | 9 | 10 | 11 | 1 | 2 | 3 | 4 |
| Liquid B | (c) | Dodecyl phosphate | part by mass | - | - | - | - | - | - | - |
| | | Hexadecyl phosphate | part by mass | - | - | - | - | - | - | - |
| | | MHP | part by mass | - | - | 30.0 | - | - | 30.0 | - |
| | | MDP | part by mass | - | - | - | 30.0 | 10.0 | - | - |
| | | M12P | part by mass | - | 30.0 | - | - | - | - | - |
| | | 4-MET | part by mass | 20.0 | - | - | - | - | - | - |
| | (d) | ZnO | part by mass | 0.3 | 1.0 | 0.4 | 0.4 | 1.0 | - | 0.4 |
| | | $ZnCl_2$ | part by mass | 2.5 | - | 2.5 | 2.5 | - | - | 2.5 |
| | | $Zn(OH)_2$ | part by mass | 0.2 | - | - | - | - | - | - |
| | (b2) | Water | part by mass | 15.0 | 12.0 | 12.0 | 12.0 | 14.0 | 12.0 | 12.0 |
| | (e) | Ethanol | part by mass | 37.0 | 58.0 | 58.0 | 53.0 | 14.0 | 58.0 | 58.0 |
| | | Isopropanol | part by mass | 20.0 | - | - | - | - | - | - |
| | | HEMA | part by mass | 8.0 | - | - | - | 28.0 | - | - |
| | | DEAA | part by mass | - | - | - | - | - | - | - |
| | | DMAEMA | part by mass | - | - | - | 5.0 | - | - | - |
| | Organic compound other than (c) and (e) | CPC | part by mass | - | 5.0 | - | 5.0 | - | - | - |
| | | MDPB | part by mass | - | - | - | - | - | - | - |
| | | Bis-GMA | part by mass | - | - | - | - | 30.0 | - | - |
| | | CQ | part by mass | - | - | - | - | 2.0 | - | - |
| | | EDMB | part by mass | - | - | - | - | 1.0 | - | - |
| | Additional component | NaF | part by mass | - | - | - | - | - | - | - |
| | | KF | part by mass | - | - | 0.1 | 0.1 | - | 0.1 | 0.1 |

(continued)

| | Kind of component | Name of component | Unit | Example | | | Comparative Example | | | |
|---|---|---|---|---|---|---|---|---|---|---|
| | | | | 9 | 10 | 11 | 1 | 2 | 3 | 4 |
| Mineral density restoration capability | | | % | 22.1 | 21.0 | 15.8 | 15.2 | 2.2 | 4.3 | 1.7 |

As shown in Table 1, Examples 1 to 11 coating the liquid A containing the components (a1) and (b1) and the liquid B containing the components (c), (d1), and (b2) and containing substantially no crosslinking polymerizable monomer on the tooth surface provided a higher mineral density restoration capability than Comparative Examples. In particular, Examples 1 to 10 coating the liquid A and the liquid B on the tooth surface in this order provided a high mineral density restoration capability exceeding 20%.

On the other hand, Comparative Example 2 using the liquid B containing a crosslinking polymerizable monomer, Comparative Example 3 using the liquid B containing no zinc ion, and Comparative Example 4 using the liquid B containing no compound (c) having an acidic group provided a significantly decreased mineral density restoration capability as compared to Examples. Comparative Example 1 using no liquid A also provided a largely decreased mineral density restoration capability as compared to Examples.

Industrial Applicability

The dental composition kit of the present invention has a high mineral density restoration capability, and achieves the restoration of an early demineralized tooth substance through a simple treatment, and therefore, can be favorably applied to the preventive treatment of a root surface caries and the restoration remedy of an early caries.

## Claims

**1.** A dental composition kit comprising a liquid A and a liquid B,

the liquid A containing a calcium ion (a1) and water (b1),
the liquid B containing a compound (c) having an acidic group represented by the following formula (I):

$$R^1\text{-}X_k \qquad \text{Formula (I)}$$

wherein in the formula (I), $R^1$ represents an organic group having 40 or less carbon atoms having or not having at least one (meth)acryloyloxy group; X represents an acidic group; and k represents an integer of 1 or 2, in which in the case where k represents 2, two acidic groups X are the same as or different from each other,
a zinc ion (d1), and water (b2), containing substantially no crosslinking polymerizable monomer.

**2.** The dental composition kit according to claim 1, wherein the liquid B further contains a hydrophilic solvent (e).

**3.** The dental composition kit according to claim 1 or 2, wherein the calcium ion (a1) is derived from at least one calcium compound (a) represented by the following formula (II):

$$(Ca^{2+})_c \cdot (Y^{n-})_d \qquad \text{Formula (II)}$$

wherein in the formula (II), $Y^{n-}$ represents an anion as a counter ion of the calcium ion; n represents an integer of 1, 2, or 3; and c and d each represent an integer of 1, 2, or 3, in which c, d, and n have a relationship satisfying the equation 2c = nd.

**4.** The dental composition kit according to claim 1 or 2, wherein the calcium ion (a1) is derived from at least one calcium compound (a) selected from the group consisting of $CaCl_2$, $CaBr_2$, $CaI_2$, $Ca(HCO_2)_2$, $Ca(HCO_3)_2$, and $Ca(CH_3COO)_2$.

**5.** The dental composition kit according to claim 1 or 2, wherein the compound (c) having an acidic group is represented by the following formula (III):

$$R^1\text{-}((A^{m-})_a \cdot (H^+)_b)_k \qquad \text{Formula (III)}$$

wherein in the formula (III), $R^1$ and k have the same meanings as defined in the formula (I); $A^{m-}$ represents an m-valent acid anionic group derived from the acidic group X; m represents an integer of 1, 2, or 3; and a and b each represent an integer of 1, 2, or 3, and have a relationship satisfying the equation am = b.

**6.** The dental composition kit according to claim 1 or 2, wherein the compound (c) having an acidic group is a compound represented by the following formula (IV):

$$R^2\text{-}R^3\text{-O-P(=O)(-OH)}_2 \qquad \text{Formula (IV)}$$

wherein in the formula (IV), $R^2$ represents a (meth)acryloyloxy group or hydrogen; and $R^3$ represents an alkylene group having 6 to 20 carbon atoms.

**7.** The dental composition kit according to claim 1 or 2, wherein the zinc ion (d1) is derived from at least one zinc compound (d) selected from the group consisting of ZnO and a compound represented by the following formula (V):

$$(Zn^{2+})_h \cdot (P^{r-})_i \qquad \text{Formula (V)}$$

wherein in the formula (V), $P^{r-}$ represents an anion as a counter ion of the zinc ion; r represents an integer of 1, 2, or 3; and h and i each represent an integer of 1, 2, or 3, in which h, i, and r have a relationship satisfying the equation 2h = ri.

**8.** The dental composition kit according to claim 1 or 2, wherein the zinc ion (d1) is derived from at least one zinc compound (d) selected from the group consisting of ZnO, $ZnCl_2$, $Zn(OH)_2$, and $ZnF_2$.

## INTERNATIONAL SEARCH REPORT

International application No.
**PCT/JP2024/022269**

**A. CLASSIFICATION OF SUBJECT MATTER**

***A61K 6/20***(2020.01)i; ***A61K 8/19***(2006.01)i; ***A61K 8/27***(2006.01)i; ***A61K 8/55***(2006.01)i; ***A61Q 11/00***(2006.01)i
FI: A61K6/20; A61K8/27; A61K8/19; A61K8/55; A61Q11/00

According to International Patent Classification (IPC) or to both national classification and IPC

**B. FIELDS SEARCHED**

Minimum documentation searched (classification system followed by classification symbols)

A61K6/20; A61K8/19; A61K8/27; A61K8/55; A61Q11/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2014-189488 A (KURARAY NORITAKE DENTAL INC.) 06 October 2014 (2014-10-06) entire text | 1-8 |
| A | JP 4-217904 A (G C DENTAL IND CORP.) 07 August 1992 (1992-08-07) entire text | 1-8 |
| A | US 2012/0315226 A1 (LEGEROS, R. Z.) 13 December 2012 (2012-12-13) whole document | 1-8 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 August 2024** | **27 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/022269**

| Patent document cited in search report | Publication date (day/month/year) | Patent family member(s) | Publication date (day/month/year) |
|---|---|---|---|
| JP 2014-189488 A | 06 October 2014 | (Family: none) | |
| JP 4-217904 A | 07 August 1992 | US 5234971 A<br>whole document | |
| US 2012/0315226 A1 | 13 December 2012 | WO 2012/135426 A2 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H03258724 A
- JP H04217904 A